(19) Europäisches Patentamt
European Patent Office
Office européen des brevets

(11) Publication number: **0 650 974 A1**

(12) **EUROPEAN PATENT APPLICATION**

(21) Application number: **93308624.1**

(22) Date of filing: **28.10.93**

(51) Int. Cl.[6]: **C07H 15/203**, C07H 15/04, A61K 31/70, C07H 15/10, C07H 15/18, C07H 15/26, C07H 17/02, C07H 17/06

(43) Date of publication of application: **03.05.95 Bulletin 95/18**

(84) Designated Contracting States: **DE FR GB IT**

(71) Applicant: **TANABE SEIYAKU CO., LTD.**
**2-10, Dosho-machi 3-chome**
**Chuo-ku**
**Osaka (JP)**

(72) Inventor: **Kurita, Hironori**
**No. 9-27, Kita-machi 2-chome,**
**Warabi-shi**
**Saitama-ken (JP)**
Inventor: **Yamagushi, Totaro**
**No. 4-7, Ryoke 7-chome, Urawa-shi**
**Saitama-ken (JP)**
Inventor: **Onta, Tokio**
**No. 29-6, Kamiishihara 2-chome, Chofu-shi**
**Tokyo-to (JP)**

(74) Representative: **Baverstock, Michael George Douglas et al**
**BOULT, WADE & TENNANT**
**27 Furnival Street**
**London, EC4A 1PO (GB)**

(54) **Acylaminosaccharide derivative and process for preparing the same.**

(57) There are disclosed an acylaminosaccharide derivative represented by the formula (I):

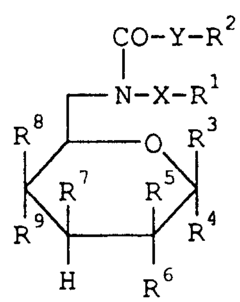

(I)

wherein $R^1$ is H, aryl or heterocyclic; X is a mere bond, alkylene, alkenylene or alkynylene; $R^2$ is (1) H, (2) aryl or heterocyclic, (3) aryl lower alkylcarbamoyl, (4) substituted carbonyl, or (5) carboxyl which may be esterified; Y is a mere bond, alkylene, alkenylene, alkynylene or tricycloalkylene; $R^3$ and $R^4$ are different and either hydrogen, or lower alkoxy or phenoxy; $R^5$ and $R^6$ are different and either hydrogen or hydroxy; and when $R^7$ is hydroxy, $R^8$ and $R^9$ are different and either hydrogen or hydroxy, or when $R^7$ and $R^8$ are bonded to each other to form lower alkylenedioxy, $R^9$ is hydrogen,
or a pharmaceutically acceptable salt thereof, and a process for preparing the same.

EP 0 650 974 A1

## BACKGROUND OF THE INVENTION

The present invention relates to a novel acylaminosaccharide derivative which has an infection-preventive activity and/or a leukocyte-increasing activity and a process for preparing the same.

It has been known that by enhancing functions in a living body of leukocytes, particularly those of neutrophils, macrophages and lymphocytes, a preventing activity of bacterial or fungal infections, an anti-tumor activity and an extensive immunological activity can be produced.

As a compound showing such an activity, particularly an infection-preventive activity, there has been known, for example, a compound in which an N-acyl-N-alkylamino group is substituted at 1-position of a saccharide (Japanese Patent Publication No. 40036/1989).

On the other hand, methyl 6-deoxy-2,3-di-O-methyl-6-methyl-acetamido-α-D-glucopyranoside is disclosed in "Carbohydrate Research", Vol. 45, pp. 65 to 72 (1975), but no pharmacological activity of said compound is disclosed.

## SUMMARY OF THE INVENTION

The present invention is to provide a novel compound having an infection-preventive activity and/or a leukocyte-increasing activity, comparable to or more excellent than those of conventionally known acylaminosaccharide derivatives.

The acylaminosaccharide derivative of the present invention is represented by the formula (I):

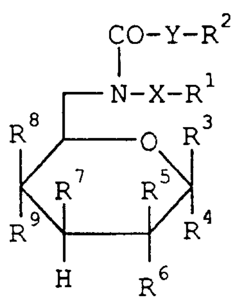

(I)

wherein $R^1$ represents hydrogen atom, or an aryl group or a heterocyclic group which may have a substituent; X represents a single bonding arm, an alkylene group, an alkenylene group or an alkynylene group; $R^2$ represents (1) hydrogen atom, (2) an aryl group or a heterocyclic group which may have a substituent, (3) an aryl lower alkylcarbamoyl group which may have a substituent, (4) carbonyl group which is substituted by a group obtained by eliminating a hydrogen atom from amino group of an amino acid or an ester thereof, or (5) carboxyl group which may be esterified; Y represents a single bonding arm, an alkylene group which may have a cycloalkylene group at an end thereof, an alkenylene group, an alkynylene group or a tricycloalkylene group; $R^3$ and $R^4$ are different from each other, and when one represents hydrogen atom, another represents a lower alkoxy group, or substituted or unsubstituted phenoxy group; $R^5$ and $R^6$ are different from each other, and represent either hydrogen atom or hydroxy group; and when $R^7$ represents hydroxy group, $R^8$ and $R^9$ are different from each other and represent either hydrogen atom or hydroxy group, or when $R^7$ and $R^8$ are bonded to each other at ends thereof to form a lower alkylenedioxy group, $R^9$ represents hydrogen atom.

The objective compound (I) of the present invention is structurally characterized by introducing an N-acyl-N-alkylamino group or an N-acyl-N-alkenylamino group at 6-position of the saccharide, and is a useful pharmaceutical compound as an infection-preventive agent or an anti-infectious property enhancing agent since it shows an excellent infection-preventive activity and/or an excellent leukocyte-increasing activity.

Also, the process for preparing the aminosaccaride compound represented by the formula (I) of the present invention comprises reacting, for example, an aminosaccharide compound represented by the formula (II):

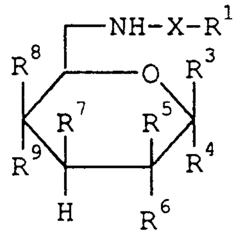

(II)

wherein the symbols have the same meanings as above, or a salt thereof, with a carboxylic acid compound represented by the formula (III):

$R^2$-Y-COOH (III)

wherein the symbols have the same meanings as above, a salt thereof, or a reactive derivative thereof.

DESCRIPTION OF THE PREFERRED EMBODIMENTS

In the following, the present invention is explained in detail.

An example of the alkylene group of the present invention may include an alkylene group having 1 to 21 carbon atoms; the alkenylene group and the alkynylene group may include an alkenylene group and an alkynylene group having 2 to 17 carbon atoms; and the cycloalkylene group may include a cycloalkylene group having 4 to 7 carbon atoms. The lower alkyl group, the lower alkylene group and the lower alkoxy group may include those having 1 to 6 carbon atoms; the lower alkylcarbamoyl group, the mono-lower alkylcarbamoyl group and the di-lower alkylcarbamoyl group may include those having 1 to 6 carbon atoms in the alkyl moiety; the lower alkylenedioxy group may include an alkylenedioxy group having 1 to 6 carbon atoms in the alkylene moiety and the lower alkanoyl group may include those having 2 to 6 carbon atoms. The alkylene group, the alkenylene group, the alkynylene group, the alkyl group and the alkoxy group may include those in the form of a straight chain or a branched chain.

An example of the aryl group of the present invention may include phenyl group, naphthyl group and tetrahydronaphthyl group; an example of the heterocyclic group may include a monocyclic or bicyclic heterocyclic group containing 1 to 2 hetero-atoms selected from nitrogen, oxygen and sulfur atoms, and specifically benzofuranyl, pyridyl, thienyl, imidazolyl, thiazolyl, oxazolyl, pyrazolyl, pyrrolyl, pyrazinyl, indolyl, quinolyl and benzothienyl groups. An example of the substituent on the aryl group, the heterocyclic group and the phenoxy group may include a lower alkyl group, a lower alkoxy group, a lower alkanoyl group, carboxyl group which may be esterified, a halogen atom, phenyl group, amino group which may be substituted by a lower alkanoyl group or a phenyl lower alkoxycarbonyl group, nitro group, a mono- or di-lower alkyl carbamoyl group, and piperidinocarbonyl group. An example of the amino acid may include a natural protein amino acid, an antipode or a racemic modification thereof, and may preferably include alanine, leucine, phenylalanine, valine, and isoleucine.

Also, the esterified carboxyl group or the esterified amino acid may include, for example, a lower alkyl ester, a phenyl lower alkyl ester and the like, and may preferably include a methyl ester, an ethyl ester and a benzyl ester.

An example of the alkylene group having a cycloalkylene group at an end thereof may include an alkylene group having a cyclohexylene group at an end thereof; and an example of the tricycloalkylene group may include a group which bonds to a neighboring $R^2$ to form adamantyl group.

A specific example of the objective compound (I) of the present invention may include a compound wherein $R^1$ is (1) hydrogen atom, (2) phenyl group which may be substituted by 1 to 3 groups selected from a lower alkyl group, a lower alkoxy group, a lower alkanoyl group, carboxyl group which may be esterified, a halogen atom, phenyl group, amino group which may be substituted by a lower alkanoyl group or a phenyl lower alkoxycarbonyl group, nitro group, a mono-or di-lower alkylcarbamoyl group and piperidinocarbonyl group, (3) naphthyl group, (4) tetrahydronaphthyl group, (5) benzofuranyl group, (6) pyridyl group or (7) thienyl group; X is a single bonding arm, a straight chain-alkylene group or a straight chain-alkenylene group; $R^2$ is (1) hydrogen atom, (2) phenyl group which may be substituted by a lower alkoxy group, (3) a phenyl lower alkylcarbamoyl group which may be substituted by 1 to 3 lower alkoxy groups, (4) carbonyl group which is substituted by a group obtained by eliminating hydrogen atom from amino group of alanine, leucine or phenylalanine, or an ester thereof, or (5) carboxyl group which may be

esterified; Y is a single bonding arm, a straight chain-alkylene group, a straight chain-alkylene group having cyclohexylene group at the end, a straight chain-alkenylene group or a straight chain-alkynylene group, or a group which bonds to the neighboring $R^2$ to form adamantyl group; $R^3$ and $R^4$ are different each other and when one is hydrogen atom, another is a lower alkoxy group or phenoxy group; $R^5$ and $R^6$ are different each other, and are hydrogen atom or hydroxy group; and when $R^7$ is hydroxy group, $R^8$ and $R^9$ are different each other and are hydrogen atom or hydroxy group, or when $R^7$ and $R^8$ are bonded to each other at ends thereof to form a lower alkylenedioxy group, $R^9$ is hydrogen atom.

Among the above compounds, a pharmaceutically preferred compound may include a compound wherein $R^1$ is (1) hydrogen atom, (2) phenyl group which may be substituted by 1 to 2 groups selected from a lower alkoxy group, carboxyl group which may be esterified, a halogen atom, and piperidinocarbonyl group, or (3) naphthyl group; X is a single bonding arm, a straight chain-alkylene group or a straight chain alkenylene group; $R^2$ is hydrogen atom or phenyl group; Y is a straight chain-alkylene group, a straight chain-alkenylene group or a straight chain-alkynylene group; $R^3$ and $R^4$ are different each other and when one is hydrogen atom, another is a lower alkoxy group; $R^5$ is hydrogen atom; $R^6$ and $R^7$ are hydroxy groups; and $R^8$ and $R^9$ are different each other and are hydrogen atom or hydroxy group.

Pharmaceutically, more preferred compound may include a compound wherein $R^1$ is hydrogen atom or phenyl group; X is a straight chain-alkylene group; $R^2$ is hydrogen atom; Y is a straight chain-alkylene group, a straight chain alkenylene group or a straight chain-alkynylene group; $R^3$, $R^5$ and $R^8$ are hydrogen atom; $R^4$ is a lower alkoxy group; and $R^6$, $R^7$ and $R^9$ are hydroxy group.

The objective compound (I) of the present invention may include an optical isomer or a mixture thereof.

The objective compound (I) of the present invention can be used for medical/pharmaceutical uses in the free form or in the form of pharmaceutically acceptable salt thereof. Such a salt may include, for example, an alkali metal salt, an alkaline earth metal salt, an amino acid salt, an organic acid salt such as succinate, maleate, tartrate and methanesulfonate, a mineral acid salt such as hydrochloride, sulfate and nitrate, and the like.

The objective compound (I) of the present invention can be orally and parenterally administered, and can be used in combination with an excipient which is suitable for oral or parenteral administration, as a pharmaceutical preparation. The pharmaceutical preparation can be a solid preparation such as tablet, capsule and powder, or a liquid preparation such as solution, suspension and emulsion. It can be also administered parenterally in the form of injection.

The dose of the objective compound (I) of the present invention, varying depending on the age, the body weight and the condition of patient, or the severity of the disease, is 1 to 500 mg/kg/day, preferably 10 to 100 mg/kg/day for the oral administration, and 0.1 to 500 mg/kg/day, preferably 1 to 100 mg/kg/day for the parenteral administration.

According to the present invention, the objective compound (I) can be manufactured by reacting, for example, an aminosaccharide compound represented by the formula (II):

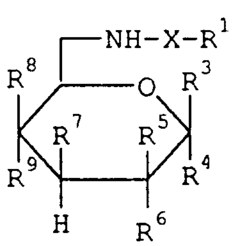

(II)

wherein the symbols have the same meanings as above, or a salt thereof, with a carboxylic acid compound represented by the formula (III):

$R^2$-Y-COOH (III)

wherein the symbols have the same meanings as above, a salt thereof, or a reactive derivative thereof.

The condensation reaction of the aminosaccharide compound (II) or the salt thereof with the carboxylic acid compound (III) or the salt thereof can be carried out in a suitable solvent in the presence of a dehydrating agent. The dehydrating agent may include, for example, dicyclohexylcarbodiimide and carbonyldiimidazole. As the salt of the aminosaccharide compound (II), for example, a mineral acid salt such as a hydrochloride, or an organic acid salt such as a fumarate, a maleate, a malate and an oxalate,

conventionally used, can be used. On the other hand, as the salt of the carboxylic acid compound (III), an alkali metal salt and an alkaline earth metal salt, conventionally used, can be used. Prior to the reaction with the aminosaccharide compound (II), the salt is preferably converted to a free carboxylic acid thereof.

The condensation reaction of the aminosaccharide compound (II) or the salt thereof with the reactive derivative of the carboxylic acid compound (III) can be carried out in a suitable solvent in the presence or the absence of an acid acceptor. As the reactive derivative, for example, an acid halide, a mixed acid anhydride, an active ester and the like which have been conventionally used for an acid-amide condensation, can be used. The acid acceptor, may include, for example, an alkali metal hydroxide, an alkali metal carbonate, an alkali metal hydrogencarbonate, a tri-lower alkylamine, an N,N-di-lower alkylaniline, pyridine, and the like.

As the solvent for the reaction, any inactive solvent can be used, as far as it does not affect the reaction in any condensation reaction, and there can be preferably mentioned, for example, trichloromethane, dichloromethane, dioxane, and tetrahydrofuran. The condensation reaction can be preferably carried out under cooling to the boiling point of the solvent, for example, at a temperature between -20 °C and 70 °C, preferably 0 °C and 20 °C.

Thus obtained objective compound (I) of the present invention can be mutually converted, if required. For example, the compound (I) wherein $R^1$ is phenyl group substituted by amino group can be also manufactured by catalytically reducing the compound (I) wherein $R^1$ is phenyl group substituted by a phenyl lower alkoxycarbonylamino group. Also, the compound (I) wherein $R^1$ is phenyl group which is substituted by a lower alkanoylamino group can be also manufactured by condensation-reacting a compound wherein $R^1$ is phenyl group substituted by amino group, with a lower alkanoyl halide.

The compound (I) wherein $R^2$ is carbonyl group which is substituted by a group obtained by eliminating hydrogen atom from amino group of an amino acid or an ester thereof, or an aryl lower alkylcarbamoyl group which may have a substituent, can be manufactured by reacting the compound (I) wherein $R^2$ is carboxyl group with the corresponding amine compound.

The above mutual conversion reaction can be carried out according to a conventional method. The catalytic reduction can be carried out, for example in the presence of palladium-carbon at an ordinary temperature and an ordinary pressure. The condensation reaction with the lower alkanoyl halide can be carried out in the presence of an acid acceptor (an alkali metal hydroxide, an alkali metal carbonate, an alkali metal hydrogencarbonate, a tri-lower alkylamine, an N,N-di-lower alkylaniline, pyridine and the like). The condensation reaction with the corresponding amine compound can be carried out in the presence of a dehydrating agent (dicyclohexylcarbodiimide, carbonyldiimidazole, and the like).

The starting compound (II) of the present invention can be manufactured by heat-reacting (for example, at 50 °C to 180 °C) a compound represented by the formula (IV):

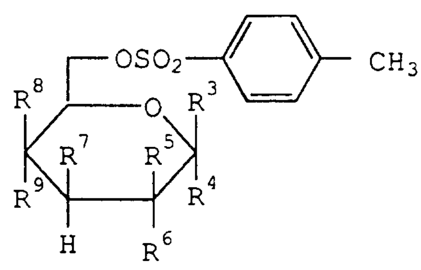

(IV)

wherein the symbols have the same meanings as above, with an amine compound represented by the formula (V):

$R^1$-X-$NH_2$ (V)

wherein the symbols have the same meanings as above, according to a conventional method, for example, in a proper solvent or without a solvent, in the presence or the absence of a reaction-accelerator (for example, sodium iodide).

## EXAMPLES

### Example 1

To 1.16 g of methyl 6-deoxy-6-(4-phenylbutyl)amino-α-D-glucopyranoside dissolved in 20 ml of tetrahydrofuran was added 0.73 g of triethylamine, and 1.3 g of octadecanoyl chloride was added to the mixture with stirring. The mixture was stirred at a room temperature overnight, and the precipitate was filtered. The filtrate was concentrated under reduced pressure, and the residue obtained was purified by silica gel column chromatography (solvent; chloroform : methanol = 15 : 1) to give 1.56 g of methyl 6-deoxy-6-[N-octadecanoyl-N-(4-phenylbutyl)amino]-α-D-glucopyranoside as a foam.

Yield: 73 %

MASS (m/z): 592

$IR^{neat}\nu_{max}(cm^{-1})$: 3350, 1640

### Examples 2 to 13

The corresponding starting compounds obtained in Reference Examples 1 to 4 and the corresponding carboxylic acid compounds were treated in the same manner as in Example 1 to give compounds shown in Table 1.

Table 1

| Example No. | 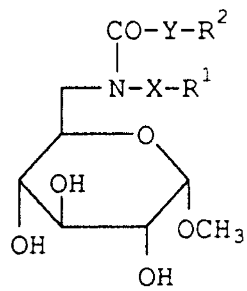 | | | |
|---|---|---|---|---|
| | $-X-R^1$ | $-Y-R^2$ | MASS (m/z) | $IR^{Nujol}\nu_{max}$ ($cm^{-1}$) |
| 2 | $-(CH_2)_4-$ 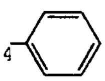 | $-(CH_2)_6-CH_3$ | 452 | 3400,1640 |
| 3 | $-(CH_2)_4-$ 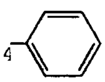 | $-(CH_2)_{10}-CH_3$ | 508 | 3380,1630 |
| 4 | $-(CH_2)_4-$ 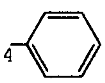 | $-(CH_2)_3-$ 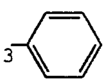 | 472 | 3400,1620 |
| 5 | $-(CH_2)_6-CH_3$ | $-(CH_2)_6-CH_3$ | 418 | 3400,1650 |
| 6 | $-(CH_2)_6-CH_3$ | $-(CH_2)_{10}-CH_3$ | 474 | 3400,1620 |
| 7 | $-(CH_2)_6-CH_3$ | $-(CH_2)_{16}-CH_3$ | 558 | 3400,1620 |
| 8 | $-(CH_2)_6-CH_3$ | $-(CH_2)_3-$ 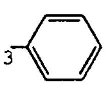 | 438 | 3400,1620* |
| 9 | $-(CH_2)_{10}-CH_3$ | $-(CH_2)_6-CH_3$ | 474 | 3400,1650* |
| 10 | $-(CH_2)_{10}-CH_3$ | $-(CH_2)_{10}-CH_3$ | 530 | 3400,1630 |
| 11 | $-(CH_2)_{10}-CH_3$ | $-(CH_2)_{16}-CH_3$ | 614 | 3350,1630* |
| 12 | $-(CH_2)_{10}-CH_3$ | $-(CH_2)_3-$ 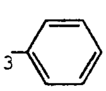 | 494 | 3400,1620 |
| 13 | $-(CH_2)_{17}-CH_3$ | $-(CH_2)_{10}-CH_3$ | 628 | 3380,1650 |

*: $IR^{neat}\nu_{max}$ ($cm^{-1}$):

Example 14

To 1.15 g of methyl 6-deoxy-6-[4-(4-methoxyphenyl)butyl]-amino-α-D-glucopyranoside dissolved in 25 ml of tetrahydrofuran were added 740 mg of potassium carbonate and 8 ml of water, and under ice-cooling and stirring, 1.08 g of octadecanoyl chloride dissolved in tetrahydrofuran was added dropwise to the

mixture, followed by stirring for 15 minutes. Further, 1 ml of methanol was added, followed by stirring for 30 minutes, and the mixture was extracted with ethyl acetate. The extracted solution was washed and dried, and the solvent was removed by distillation. The residue obtained was purified by silica gel column chromatography (solvent; chloroform : methanol = 20 : 1) to give 1.91 g of methyl 6-deoxy-6-{N-octadecanoyl-N-[4-(4-methoxyphenyl)butyl]amino}-α-D-glucopyranoside as an oily product.

Yield: 95 %

MASS (m/z): 622

$IR^{neat}\nu_{max}$ ($cm^{-1}$): 3400, 1620

Examples 15 to 63

The corresponding starting compounds obtained in Reference Examples 1 and 3 to 38 and the corresponding carboxylic acid compounds were treated in the same manner as in Example 14 to give compounds shown in Table 2.

Table 2

| Example No. | 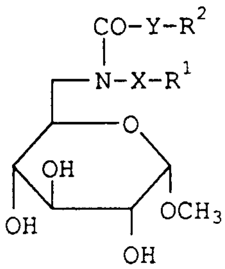 | | | |
|---|---|---|---|---|
| | $-X-R^1$ | $-Y-R^2$ | MASS (m/z) | $IR^{Neat}\nu_{max}$ ($cm^{-1}$) |
| 15 | $-(CH_2)_4$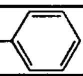 | $-CH_3$ | 368 | 3380,1620 |
| 16 | $-(CH_2)_4$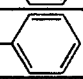 | $-(CH_2)_2-CH_3$ | 396 | 3380,1620 |
| 17 | $-(CH_2)_4$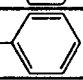 | $-(CH_2)_5-CH_3$ | 438 | 3380,1620 |
| 18 | $-(CH_2)_4$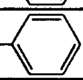 | $-(CH_2)_7-CH_3$ | 466 | 3380,1620 |
| 19 | $-(CH_2)_4$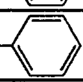 | $-(CH_2)_9-CH_3$ | 494 | 3380,1620 |
| 20 | $-(CH_2)_4$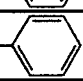 | $-(CH_2)_{12}-CH_3$ | 536 | 3400,1620 |
| 21 | $-(CH_2)_4$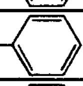 | $-(CH_2)_{13}-CH_3$ | 550 | 3350,1620 |
| 22 | $-(CH_2)_4$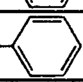 | $-(CH_2)_{14}-CH_3$ | 564 | 3350,1620 |
| 23 | $-(CH_2)_4$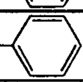 | $-(CH_2)_{15}-CH_3$ | 578 | 3350,1620 |
| 24 | $-(CH_2)_4$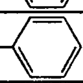 | $-(CH_2)_{18}-CH_3$ | 620 | 3400,1620 |
| 25 | $-(CH_2)_4$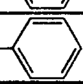 | $-(CH_2)_{20}-CH_3$ | 648 | 3400,1620 |
| 26 | $-(CH_2)_4$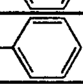 | $-(CH_2)_4-COOCH_3$ | 468 | 3400,1740, 1620 |
| 27 | $-(CH_2)_4$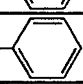 | $-(CH_2)_8-CH=CH_2$ | 492 | 3400,1620 |
| 28 | $-(CH_2)_4$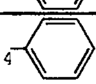 | $-(CH_2)_7CH=CH-(CH_2)_7CH_3$ | 590 | 3400,1620 |
| 29 | $-(CH_2)_{10}-CH_3$ | $-(CH_2)_4-COOCH_3$ | 490 | 3400,1740, 1620 |
| 30 | $-(CH_2)_{17}-CH_3$ | $-(CH_2)_3$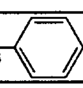 | 592 | 3400,1620 |

Table 2 (Contd.)

| Example No. | 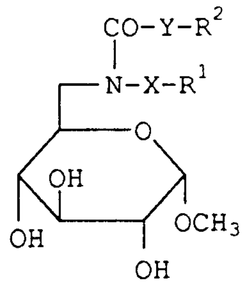 | | | |
|---|---|---|---|---|
| | $-X-R^1$ | $-Y-R^2$ | MASS (m/z) | $IR^{Neat}\upsilon_{max}$ ($cm^{-1}$) |
| 31 | 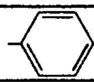 | $-(CH_2)_{16}-CH_3$ | 536 | 3400,1660 |
| 32 | 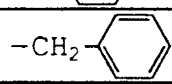 | $-(CH_2)_{16}-CH_3$ | 550 | 3390,1640 |
| 33 | 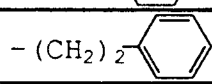 | $-(CH_2)_{16}-CH_3$ | 564 | 3400,1620 |
| 34 | 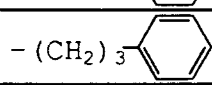 | $-(CH_2)_{16}-CH_3$ | 578 | 3400,1620 |
| 35 | 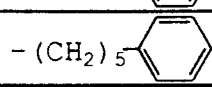 | $-(CH_2)_{16}-CH_3$ | 606 | 3400,1620 |
| 36 | 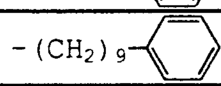 | $-(CH_2)_{16}-CH_3$ | 662 | 3400,1620 |
| 37 | 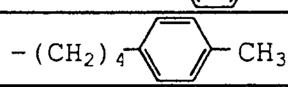 | $-(CH_2)_{16}-CH_3$ | 606 | 3380,1620 |
| 38 | 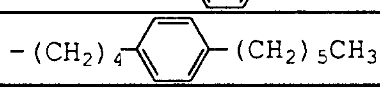 | $-(CH_2)_{16}-CH_3$ | 676 | 3380,1620 |
| 39 | 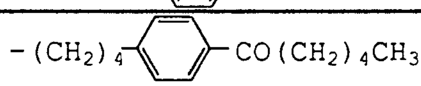 | $-(CH_2)_{16}-CH_3$ | 690 | 3390,1685 1610 |
| 40 | 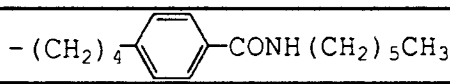 | $-(CH_2)_{16}-CH_3$ | 719 | 3340,1630 |
| 41 | 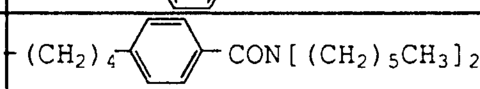 | $-(CH_2)_{16}-CH_3$ | 803 | 3390,1640 1610 |
| 42 | 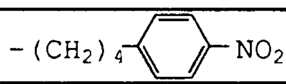 | $-(CH_2)_{16}-CH_3$ | 637 | 3390,1620 |
| 43 | 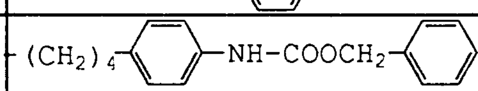 | $-(CH_2)_{16}-CH_3$ | 741 | 3400,1730 1620 |
| 44 | 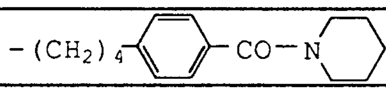 | $-(CH_2)_{16}-CH_3$ | 703 | 3380,1630 |

Table 2 (Contd.)

| Example No. | 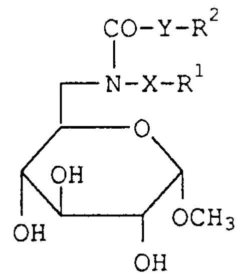 | | | |
|---|---|---|---|---|
| | $-X-R^1$ | $-Y-R^2$ | MASS (m/z) | $IR^{Neat}\upsilon_{max}$ ($cm^{-1}$) |
| 45 | 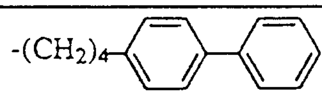 | $-(CH_2)_{16}-CH_3$ | 668 | 3400, 1620 |
| 46 | 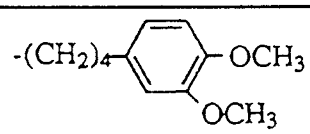 | $-(CH_2)_{16}-CH_3$ | 652 | 3390, 1625 |
| 47 | 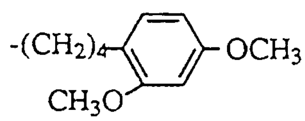 | $-(CH_2)_{16}-CH_3$ | 652 | 3380, 1620 |
| 48 | 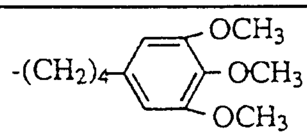 | $-(CH_2)_{16}-CH_3$ | 683 | 3400, 1620 |
| 49 | 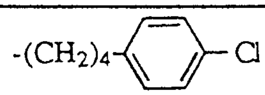 | $-(CH_2)_{16}-CH_3$ | 626 | 3390, 1635 |
| 50 | 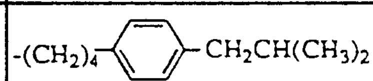 | $-(CH_2)_{16}-CH_3$ | 648 | 3400, 1622 |
| 51 | 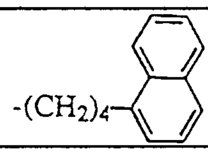 | $-(CH_2)_{16}-CH_3$ | 642 | 3380, 1620 |
| 52 | 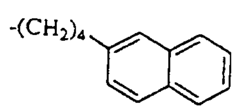 | $-(CH_2)_{16}-CH_3$ | 642 | 3380, 1620 |
| 53 | 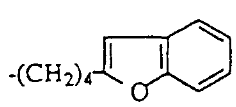 | $-(CH_2)_{16}-CH_3$ | 654 | 3400, 1620 |

Table 2 (Contd.)

| Example No. | 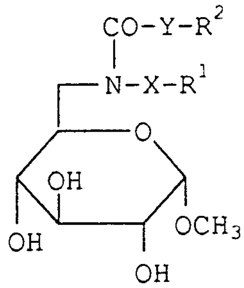 | | | |
|---|---|---|---|---|
| | $-X-R^1$ | $-Y-R^2$ | MASS (m/z) | $IR^{Neat}\upsilon_{max}$ ($cm^{-1}$) |
| 54 | 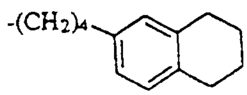 | $-(CH_2)_{16}-CH_3$ | 646 | 3390,1625 |
| 55 | 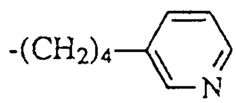 | $-(CH_2)_{16}-CH_3$ | 615 | 3400,1640 |
| 56 | 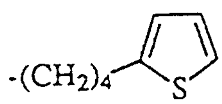 | $-(CH_2)_{16}-CH_3$ | 598 | 3400,1620 |
| 57 | 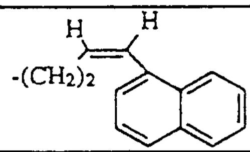 | $-(CH_2)_{16}-CH_3$ | 640 | 3380,1620 |
| 58 | 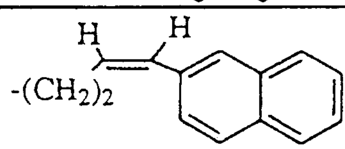 | $-(CH_2)_{16}-CH_3$ | 640 | 3380,1625 |
| 59 | 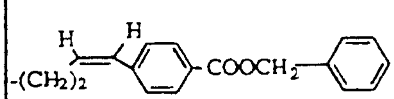 | $-(CH_2)_{16}-CH_3$ | 724 | 3390,1720, 1620 |

Table 2 (Contd)

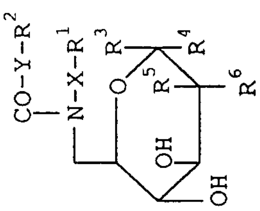

| Example No. | -X-R¹ | -Y-R² | R³ | R⁴ | R⁵ | R⁶ | MASS (m/z) | $IR^{Neat}\nu_{max}$ ($cm^{-1}$) |
|---|---|---|---|---|---|---|---|---|
| 60 | $-(CH_2)_4-$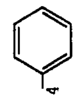 | $-(CH_2)_{16}-CH_3$ | -H | $-OCH_3$ | -OH | -H | 592 | 3400, 1620 |
| 61 | $-(CH_2)_4-$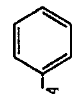 | $-(CH_2)_{16}-CH_3$ | -O-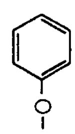 | -H | -H | -OH | 654 | 3400, 1640 |
| 62 | $-(CH_2)_4-$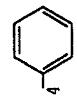 | $-(CH_2)_{16}-CH_3$ | $-O(CH_2)_3CH_3$ | -H | -H | -OH | 634 | 3520, 1620 |
| 63 | $-(CH_2)_4-$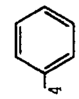 | $-(CH_2)_{16}-CH_3$ | $-OCH_3$ | -H | -H | -OH | 614 | 3300, 1620 |

Example 64

2 ml of a 10 % sodium hydroxide aqueous solution was added dropwise to 0.5 g of methyl 6-deoxy-6-[N-(5-methoxycarbonylpentanoyl)-N-undecylamino]-α-D-glucopyranoside dissolved in 20 ml of methanol

under ice-cooling, followed by stirring at a room temperature overnight. The reaction solution was concentrated under reduced pressure, and the concentrated solution was purified by a column chromatography (solvent; methanol : water = 20:70, 50:50 and 70:30) using SP-207 (High Porous Polymer, manufactured by Mitsubishi Kasei Corporation) to give 0.25 g of sodium salt of methyl 6-deoxy-6-[N-(5-carboxypentanoyl)-N-undecylamino]-α-D-glucopyranoside as a powder.

MASS (m/z): 520

$IR^{nujol}\nu_{max}(cm^{-1})$: 3300, 1640

Example 65

Methyl 6-deoxy-6-[N-(5-methoxycarbonylpentanoyl)-N-(4-phenylbutyl)amino]-α-D-glucopyranoside was treated in the same manner as in Example 64 to give sodium salt of methyl 6-deoxy-6-[N-(5-carboxypentanoyl)-N-(4-phenylbutyl)amino]-α-D-glucopyranoside as a powder.

Yield: 56 %

MASS (m/z): 498

$IR^{nujol}\nu_{max}(cm^{-1})$: 3300, 1640

Example 66

(1) 1.5 ml of a 10 % sodium hydroxide aqueous solution was added dropwise to 0.5 g of methyl 6-deoxy-6-[N-(5-methoxy-carbonylpentanoyl)-N-(4-phenylbutyl)amino]-α-D-glucopyranoside dissolved in 10 ml of methanol under ice-cooling, followed by stirring at a room temperature overnight. Methanol was removed by distillation under reduced pressure, and the residue was adjusted to pH 3 with a 10 % hydrochloric acid aqueous solution and extracted with 30 ml of chloroform. The extract was washed and dried, then the solvent was removed by distillation to give 0.38 g of methyl 6-deoxy-6-[N-(5-carboxypentanoyl)-N-(4-phenylbutyl)-amino]-α-D-glucopyranoside as an oily product.

Yield: 78 %

MASS (m/z): 454

$IR^{neat}\nu_{max}(cm^{-1})$: 3300, 1710, 1610

(2) To 0.68 g of the resulting compound dissolved in 15 ml of tetrahydrofuran were added 0.34 g of dicyclohexylcarbodiimide and 0.22 g of 1-hydroxybenzotriazole, followed by stirring at -10 °C for 10 minutes. Then, 0.31 g of hydrochloride of L-alanine methyl ester and 0.23 g of triethylamine were added to the mixture. The mixture was stirred at room temperature for 20 hours, and the precipitate formed was removed by filtration. The filtrate was concentrated under reduced pressure, and the residue obtained was purified by silica gel column chromatography (solvent; chloroform : methanol = 10 : 1) to 0.5 g of methyl 6-deoxy-6-{N-[((S)-1-methoxycarbonylethyl)aminocarbonyl]pentanoylN-(4-phenylbutyl)amino}-α-D-glucopyranoside as a foam.

Yield: 62 %

MASS (m/z): 539

$IR^{neat}\nu_{max}(cm^{-1})$: 3300, 1750, 1620

Examples 67 to 71

Methyl 6-deoxy-6-[N-(5-carboxypentanoyl)-N-(4-phenylbutyl)-amino]-α-D-glucopyranoside and the corresponding amine compound were treated in the same manner as in Example 66 (2) to give compounds shown in Table 3.

Table 3

| Example No. | 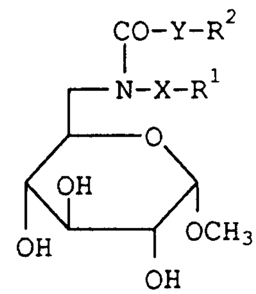 | | | |
|---|---|---|---|---|
| | $-X-R^1$ | $-Y-R^2$ | MASS (m/z) | $IR^{Neat}\nu_{max}$ ($cm^{-1}$) |
| 67 | 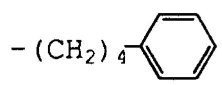 | 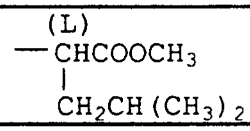 | 581 | 3300, 1740, 1620 |
| 68 | 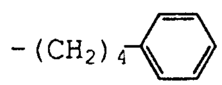 | 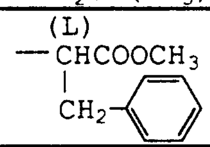 | 615 | 3400, 1740, 1620 |
| 69 | 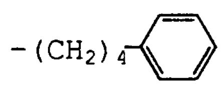 | 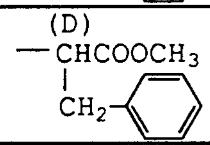 | 615 | 3400, 1740, 1620 |
| 70 | 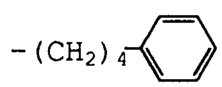 | 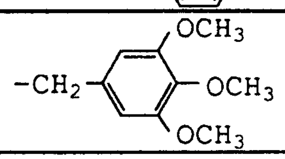 | 633 | 3350, 1620 |
| 71 | 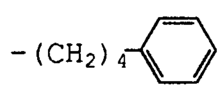 | 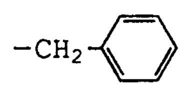 | 543 | 3300, 1610 |

Example 72

Methyl 6-deoxy-6-(4-phenylbutyl)amino-3,4-isopropylidene-β-D-galactopyranose and octadecanoyl chloride were treated in the same manner as in Example 14 to give methyl 6-deoxy-6-[N-octadecanoyl-N-(4-phenylbutyl)amino]-3,4-isopropylidene-β-D-galactopyranose as an oily product.

MASS (m/z): 632

$IR^{neat}\nu_{max}(cm^{-1})$: 3400, 1640

Example 73

Methyl 6-deoxy-6-(4-phenylbutyl)amino-β-D-galactopyranoseand octadecanoyl chloride were treated in the same manner as in Example 14 to give methyl 6-deoxy-6-[N-octadecanoyl-N-(4-phenylbutyl)amino]-β-D-galactopyranose as an oily product.

Yield: 86 %

MASS (m/z): 592

$IR^{neat}\nu_{max}(cm^{-1})$: 3400, 1640

Example 74

900 mg of a 10 % palladium-carbon was added to 1.96 g of methyl 6-deoxy-6-{N-octadecanoyl-N-[4-(4-benzyloxycarbonylphenyl)-3-butenyl]amino}-α-D-glucopyranoside dissolved in a mixture of 15 ml of tetrahydrofuran and 5 ml of ethanol, and subjected to catalytic reduction at a room temperature and an ordinary pressure. After 3 hours, the catalyst was removed by filtration, and the filtrate was concentrated under reduced pressure. The residue obtained was purified by silica gel column chromatography (solvent; chloroform : methanol = 9 : 1) to give methyl 6-deoxy-6-{N-octadecanoyl-N-[4-(4-carboxyphenyl)butyl]-amino}-α-D-glucopyranoside as an oily product.

Yield: 82 %

MASS (m/z): 636

$IR^{neat}\nu_{max}(cm^{-1})$: 3380, 1715, 1610

Example 75

(1) Methyl 6-deoxy-6-{N-octadecanoyl-N-[4-(4-benzyloxy-carbonylaminophenyl)butyl]amino}-α-D-glucopyranoside was treated in the same manner as in Example 74 to give methyl 6-deoxy-6-{N-octadecanoyl-N-[4-(4-aminophenyl)butyl]amino}-α-D-glucopyranoside as an oily product.

Yield: 97 %

MASS (m/z): 607

$IR^{neat}\nu_{max}(cm^{-1})$: 3360, 1630

(2) To 400 mg of the resulting compound dissolved in 20 ml of tetrahydrofuran were added 150 mg of potassium carbonate and 4 ml of water, and 100 mg of hexanoyl chloride dissolved in 3 ml of tetrahydrofuran was added dropwise under stirring and ice-cooling. After one hour, 2 ml of methanol was added, followed by stirring for one hour. Then, the solvent was removed by distillation, and the residue was extracted with ethyl acetate. The extract was washed and dried, then the solvent was removed by distillation. The residue obtained was purified by silica gel column chromatography (solvent; chloroform : methanol = 10 : 1) to give 458 mg of methyl 6-deoxy-6-{N-octadecanoyl-N-[4-(4-n-hexanoylaminophenyl)butyl]amino}-α-D-glucopyranoside as an oily product.

Yield: 98 %

MASS (m/z): 705

$IR^{neat}\nu_{max}(cm^{-1})$: 3400, 1660, 1620

Examples 76 to 78

Methyl 6-deoxy-6-(4-phenylbutyl)amino-α-D-glucopyranosideand the corresponding carboxylic acid compound were treated in the same manner as in Example 14 to give compounds shown in Table 4.

Table 4

| Example No. | 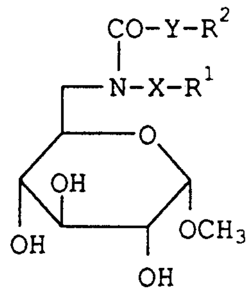 | | | |
|---|---|---|---|---|
| | $-X-R^1$ | $-Y-R^2$ | MASS (m/z) | $IR^{Nujol}\nu_{max}$ ($cm^{-1}$) |
| 76 | $-(CH_2)_4$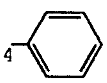 | $-(CH_2)_8C{\equiv}CH$ | 490 | 3400,1620* |
| 77 | $-(CH_2)_4$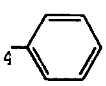 | 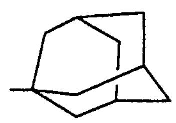 | 488 | 3350,1620 |
| 78 | $-(CH_2)_4$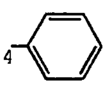 | 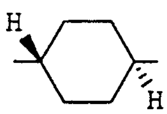 $(CH_2)_4CH_3$ | 506 | 3400,1620* |

*: $IR^{Neat}\nu_{max}$ ($cm^{-1}$)

Examples 79 to 95

The corresponding starting compounds obtained in Reference examples Nos. 1, 2, 4, and 41 to 43 and the corresponding carboxylic acid compounds were treated in the same manner as in Example 14 to give compounds shown in Table 5.

Table 5

| Example No. | 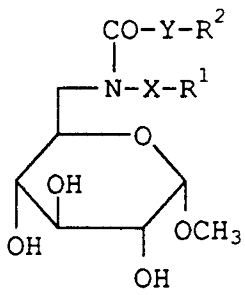 | | | |
|---|---|---|---|---|
| | $-X-R^1$ | $-Y-R^2$ | MASS (m/z) | $IR^{Neat}\nu_{max}$ ($cm^{-1}$) |
| 79 | $-(CH_2)_{17}-CH_3$ | $-(CH_2)_8-CH=CH_2$ | 612 | 3400, 1620 |
| 80 | $-(CH_2)_{17}-CH_3$ | $-(CH_2)_6-CH_3$ | 572 | 3400, 1620 |
| 81 | $-(CH_2)_{17}-CH_3$ | $-(CH_2)_9-CH_3$ | 614 | 3400, 1620 |
| 82 | $-(CH_2)_{17}-CH_3$ | $-(CH_2)_8-C\equiv CH$ | 610 | 3400, 1620 |
| 83 | $-(CH_2)_{17}-CH_3$ | $-(CH_2)_{11}-CH_3$ | 642 | 3400, 1620 |
| 84 | $-(CH_2)_{17}-CH_3$ | $-(CH_2)_{12}-CH_3$ | 656 | 3400, 1650 |
| 85 | $-(CH_2)_{17}-CH_3$ | $-(CH_2)_{16}-CH_3$ | 712 | 3400, 1640 |
| 86 | $-(CH_2)_{17}-CH_3$ | $-(CH_2)_{14}-CH_3$ | 684 | 3380, 1620 |
| 87 | $-(CH_2)_{15}-CH_3$ | $-(CH_2)_{10}-CH_3$ | 600 | 3400, 1620 |
| 88 | $-(CH_2)_{16}-CH_3$ | $-(CH_2)_{10}-CH_3$ | 614 | 3400, 1620 |
| 89 | $-(CH_2)_{18}-CH_3$ | $-(CH_2)_{10}-CH_3$ | 642 | 3400, 1640 |
| 90 | $-(CH_2)_6-CH_3$ | $-(CH_2)_{17}-CH_3$ | 572 | 3400, 1620 |
| 91 | $-(CH_2)_{17}-CH_3$ | $-(CH_2)_8-CH_3$ | 600 | 3380, 1620 |
| 92 | $-(CH_2)_{17}-CH_3$ | $-(CH_2)_3-CH_3$ | 530 | 3380, 1620 |
| 93 | $-(CH_2)_6-CH_3$ | $-(CH_2)_{15}-CH_3$ | 544 | 3400, 1640 |
| 94 | $-(CH_2)_6-CH_3$ | $-(CH_2)_{18}-CH_3$ | 586 | 3400, 1620 |
| 95 | $-(CH_2)_4$ 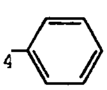 | 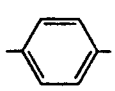 $O-(CH_2)_3-CH_3$ | 502 | 3400, 1620 |

Reference example 1

To 15 g of methyl 6-tosyl-α-D-glucopyranoside dissolved in 70 ml of dimethylformamide, 19.3 g of 4-phenylbutylamine was added, and the mixture was reacted at 90 °C for 7 hours. After the reaction, the mixture was concentrated under reduced pressure, and the residue obtained was purified by silica gel column chromatography (solvent; chloroform : methanol = 15 : 1 to 10 : 1) to give 9.71 g of methyl 6-deoxy-6-(4-phenylbutyl)amino-α-D-glucopyranosideas a foam.

Yield: 69 %

MASS (m/z): 326

$IR^{neat}\nu_{max}$($cm^{-1}$): 3350

Reference examples 2 to 4

The corresponding starting compounds were treated in the same manner as in Reference example 1 to give compounds shown in Table 6.

Table 6

| Reference example No. | 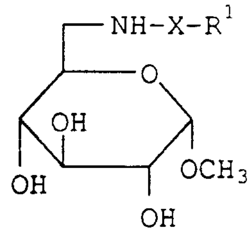 | | |
|---|---|---|---|
| | $-X-R^1$ | MASS (m/z) | $IR^{Nujol}\nu_{max}$ ($cm^{-1}$) |
| 2 | $-(CH_2)_6CH_3$ | 292 | 3400 |
| 3 | $-(CH_2)_{10}CH_3$ | 348 | 3400 |
| 4 | $-(CH_2)_{17}CH_3$ | 446 | 3400 |

Reference example 5

To 1.5 g of methyl 6-tosyl-α-D-glucopyranoside dissolved in 30 ml of toluene was added 2.7 g of 4-(4-methoxyphenyl)-butylamine, and the mixture was refluxed for 15 hours. The reaction mixture was concentrated under reduced pressure, and the residue obtained was purified by silica gel column chromatography (solvent; chloroform : methanol : aqueous ammonia = 20 : 5 : 1) to give 1.21 g of methyl 6-deoxy-6-[4-(4-methoxyphenyl)butyl]amino-α-D-glucopyranoside as an oily product.

Yield: 79 %

MASS (m/z): 356

$IR^{neat}\nu_{max}$($cm^{-1}$): 3400

Reference examples 6 to 38

The corresponding starting compounds were treated in the same manner as in Reference example 5 to give compounds shown in Table 7.

Table 7

| Reference example No. | 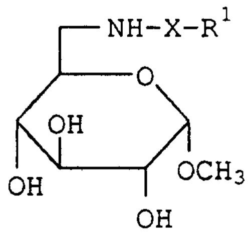 | | |
|---|---|---|---|
| | $-X-R^1$ | MASS (m/z) | $IR^{Neat}\nu_{max}$ ($cm^{-1}$) |
| 6 | 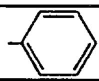 | 270 | 3390 |
| 7 | $-CH_2-$ 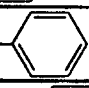 | 284 | 3390 |
| 8 | $-(CH_2)_2-$ 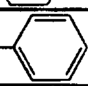 | 298 | 3400 |
| 9 | $-(CH_2)_3-$ 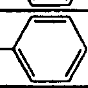 | 312 | 3300 |
| 10 | $-(CH_2)_5-$ 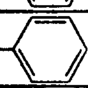 | 340 | 3360 |
| 11 | $-(CH_2)_9-$ 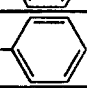 | 396 | 3390 |
| 12 | $-(CH_2)_4-$ 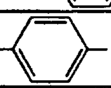 $-CH_3$ | 340 | 3400 |
| 13 | $-(CH_2)_4-$ 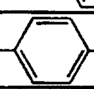 $-(CH_2)_5CH_3$ | 410 | 3380 |
| 14 | $-(CH_2)_4-$ 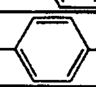 $-CO(CH_2)_4CH_3$ | 424 | 3400, 1680 |
| 15 | $-(CH_2)_4-$ 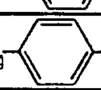 $-CONH(CH_2)_5CH_3$ | 453 | 3320, 1640 |
| 16 | $-(CH_2)_4-$ 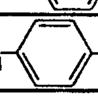 $-CON[(CH_2)_5CH_3]_2$ | 537 | 3400, 1640 |
| 17 | $-(CH_2)_4-$ 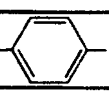 $-NO_2$ | 371 | 3380 |
| 18 | $-(CH_2)_4-$ 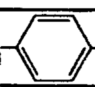 $-NH-COOCH_2-$ 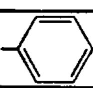 | 475 | 3300, 1705 |
| 19 | $-(CH_2)_4-$ 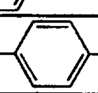 $-CO-$ 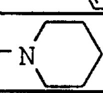 | 437 | 3380, 1630 |
| 20 | $-(CH_2)_4-$ 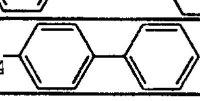 | 402 | 3400 |

Table 7 (Contd)

| Reference example No. | 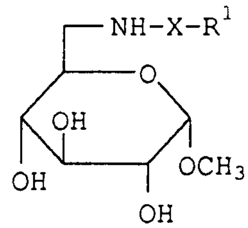 | | |
|---|---|---|---|
| | $-X-R^1$ | MASS (m/z) | $IR^{Neat}\nu_{max}$ ($cm^{-1}$) |
| 21 | 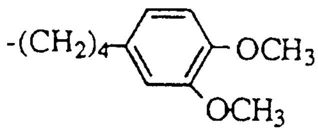 | 386 | 3380 |
| 22 | 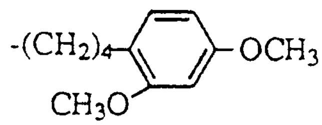 | 386 | 3390 |
| 23 | 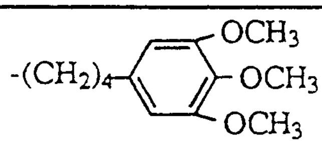 | 416 | 3400 |
| 24 | 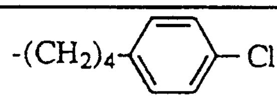 | 360 | 3390 |
| 25 | 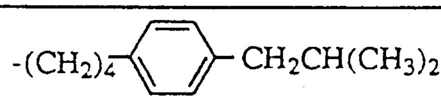 | 382 | 3400 |
| 26 | 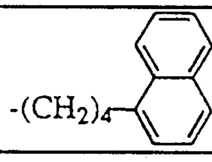 | 376 | 3380 |
| 27 | 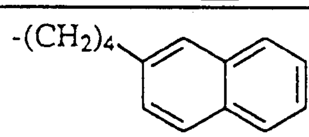 | 376 | 3380 |
| 28 | 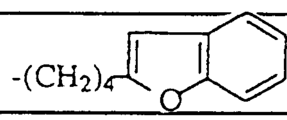 | 366 | 3400 |
| 29 | 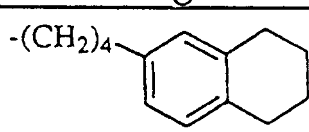 | 380 | 3380 |

Table 7 (Contd)

| Reference example No. | 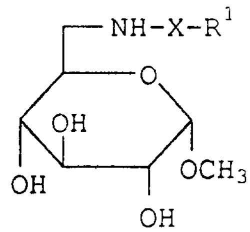 | | |
|---|---|---|---|
| | $-X-R^1$ | MASS (m/z) | $IR^{Neat}\nu_{max}$ ($cm^{-1}$) |
| 30 | 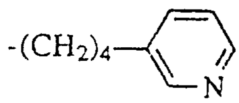 | 327 | 3350 |
| 31 | 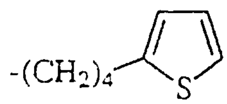 | 332 | 3400 |
| 32 | 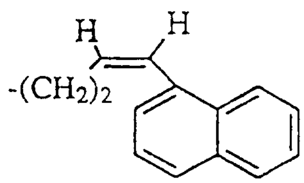 | 374 | 3360 |
| 33 | 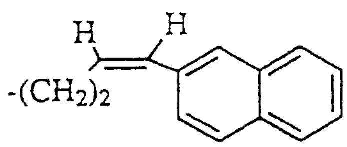 | 374 | 3380 |
| 34 | 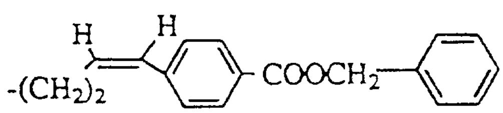 | 458 | 3380, 1720 |

Table 7 (Contd)

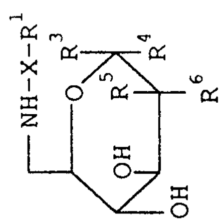

| Reference example No. | $-X-R^1$ | $R^3$ | $R^4$ | $R^5$ | $R^6$ | MASS (m/z) | $IR^{Nujol}\nu_{max}$ ($cm^{-1}$) |
|---|---|---|---|---|---|---|---|
| 35 | $-(CH_2)_4-$ 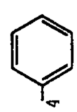 | -H | $-OCH_3$ | -OH | -H | 326 | 3400* |
| 36 | $-(CH_2)_4-$ 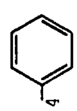 | -O- 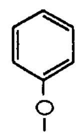 | -H | -H | -OH | 388 | 3350 |
| 37 | $-(CH_2)_4-$ 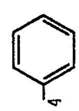 | $-O(CH_2)_3CH_3$ | -H | -H | -OH | 368 | 3350* |
| 38 | $-(CH_2)_4-$ 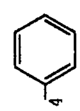 | $-OCH_3$ | -H | -H | -OH | 326 | 3300* |

*: $IR^{Neat}\nu_{max}$ ($cm^{-1}$)

Reference example 39

Methyl 6-tosyl-3,4-isopropylidene-β-D-glucopyranoside was treated in the same manner as in Reference example 5 to give methyl 6-deoxy-6-(4-phenylbutyl)amino-3,4-isopropyl-idene-β-D-glucopyranoside as an

oily product.

Yield: 95 %

MASS (m/z): 366

$IR^{neat}\nu_{max}(cm^{-1})$: 3180

Reference example 40

11 ml of a 10 % hydrochloric acid solution was added to 3.0 g of methyl 6-deoxy-6-(4-phenylbutyl)-amino-3,4-isopropyl-idene-β-D-galactopyranose dissolved in 50 ml of methanol, followed by stirring at a room temperature for 4 hours. The mixture was adjusted to pH 8 with a 10 % sodium hydroxide aqueous solution, and concentrated under reduced pressure. The residue obtained was purified by silica gel column chromatography (solvent; chloroform : methanol : aqueous ammonia = 10 : 2 : 0.2) to give 1.9 g of methyl 6-deoxy-6-(4-phenylbutyl)amino-β-D-galactopyranose as an oily product.

Yield: 71 %

MASS (m/z): 326

$IR^{neat}\nu_{max}(cm^{-1})$: 3350

Reference examples 41 to 43

The corresponding starting compounds were treated in the same manner as in Reference example 5 to give compounds shown in Table 8.

Table 8

| Reference example No. | 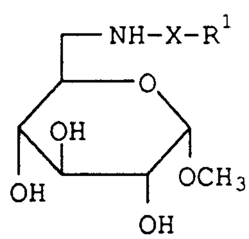 | | |
|---|---|---|---|
| | $-X-R^1$ | MASS (m/z) | $IR^{Neat}\nu_{max}$ ($cm^{-1}$) |
| 41 | $-(CH_2)_{15}CH_3$ | 418 | 3400 |
| 42 | $-(CH_2)_{16}CH_3$ | 432 | 3380 |
| 43 | $-(CH_2)_{18}CH_3$ | 460 | 3360 |

The acylaminosaccharide derivative (I), the objective compound of the present invention, is structurally characterized in that an acylamino group is introduced at 6-position of the saccharide, and is a useful pharmaceutical compound as an infection-preventive agent or an anti-infectious property enhancing agent since it has an excellent infection-preventive activity.

Concretely, it, exhibiting a bacterial or fungal infection-preventive activity and/or an anti-tumor activity, is useful for general prevention of an infectious disease in a human or an animal, and furthermore can be preferably used in congenital or acquired immunodeficiency, particularly in acquired immunodeficiency caused by severe temporary disorders after radiotherapy or treatment with a material having an immunosuppressive activity. The compound of the present invention also has a leukocyte-increasing activity. Since the compound of the present invention compensates the immunodeficiency, it can be preferably administered in combination with an anti-infectious or anti-tumor antibiotic or the other chemotherapeutic agent, or in combination with the other medicine.

## Claims

1. An acylaminosaccharide compound represented by the formula (I):

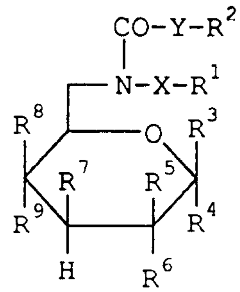

(I)

wherein $R^1$ represents hydrogen atom, or an aryl group or a heterocyclic group which may have a substituent; X represents a single bonding arm, an alkylene group, an alkenylene group or an alkynylene group; $R^2$ represents (1) hydrogen atom, (2) an aryl group or a heterocyclic group which may have a substituent, (3) an aryl lower alkylcarbamoyl group which may have a substituent, (4) carbonyl group which is substituted by a group obtained by eliminating a hydrogen atom from amino group of an amino acid or an ester thereof, or (5) carboxyl group which may be esterified; Y represents a single bonding arm, an alkylene group which may have a cycloalkylene group at an end thereof, an alkenylene group, an alkynylene group or a tricycloalkylene group; $R^3$ and $R^4$ are different from each other, and when one represents hydrogen atom, another represents a lower alkoxy group, or substituted or unsubstituted phenoxy group; $R^5$ and $R^6$ are different from each other, and represent either hydrogen atom or hydroxy group; and when $R^7$ represents hydroxy group, $R^8$ and $R^9$ are different from each other and represent either hydrogen atom or hydroxy group, or when $R^7$ and $R^8$ are bonded to each other at ends thereof to form a lower alkylenedioxy group, $R^9$ represents hydrogen atom,
or a pharmaceutically acceptable salt thereof.

2. The compound according to Claim 1, wherein $R^1$ is hydrogen atom a substituted or unsubstituted aryl group or a substituted or unsubstituted heterocyclic group; X is a single bonding arm, a $C_{1-21}$ alkylene group, a $C_{2-17}$ alkenylene group or a $C_{2-17}$ alkynylene group; $R^2$ is (1) hydrogen atom, (2) a substituted or unsubstituted aryl group or a substituted or unsubstituted heterocyclic group, (3) a substituted or unsubstituted aryl-$C_{1-6}$ alkyl-carbamoyl group, (4) carbonyl group substituted by a group obtained by eliminating a hydrogen atom from amino group of an amino acid or an ester thereof, or (5) carboxyl group which may be esterified; Y is a single bonding arm, a $C_{1-21}$ alkylene group which may have a cycloalkylene group at an end thereof, a $C_{2-17}$ alkenylene group, a $C_{2-17}$ alkynylene group or a tricycloalkylene group; $R^3$ and $R^4$ are different from each other, and when one is hydrogen atom, another is a $C_{1-6}$ alkoxy group, or a substituted or unsubstituted phenoxy group; $R^5$ and $R^6$ are different from each other, and are either hydrogen atom or hydroxy group; and when $R^7$ is hydroxy group, $R^8$ and $R^9$ are different from each other and are either hydrogen atom or hydroxy group, or when $R^7$ and $R^8$ are bonded to each other at the ends thereof to form a $C_{1-6}$ alkylenedioxy group, $R^9$ is hydrogen atom,

3. The compound according to Claim 1, wherein $R^1$ is (1) hydrogen atom, (2) phenyl group which may be substituted by 1 to 3 groups selected from a lower alkyl group, a lower alkoxy group, a lower alkanoyl group, carboxyl group which may be esterified, a halogen atom, phenyl group, amino group which may be substituted by a lower alkanoyl group or a phenyl lower alkoxycarbonyl group, nitro group, a mono- or di-lower alkylcarbamoyl group and piperidinocarbonyl group, (3) naphthyl group, (4) tetrahydronaphthyl group, (5) benzofuranyl group, (6) pyridyl group, (7) thienyl group, (8) imidazolyl group, (9) thiazolyl group, (10) oxazolyl group, (11) pyrazolyl group, (12) pyrolyl group, (13) pyradinyl group, (14) indolyl group, (15) quinolyl group or (16) benzothienyl group; X is a single bonding arm, a straight alkylene group or a straight alkenylene group; $R^2$ is (1) hydrogen atom, (2) phenyl group which may be substituted by a lower alkoxy group, (3) a phenyl lower alkylcarbamoyl group which may be substituted by 1 to 3 lower alkoxy groups, (4) carbonyl group which is substituted by a group obtained by eliminating hydrogen atom from amino group of alanine, leucine or phenylalanine, or an ester thereof,

or (5) carboxyl group which may be esterified.

4. The compound according to Claim 3, wherein $R^1$ is (1) hydrogen atom, (2) phenyl group which may be substituted by 1 or 2 groups selected from a lower alkoxy group, carboxyl group which may be esterified, a halogen atom and piperidinocarbonyl group, or (3) naphthyl group; X is a single bonding arm, a straight alkylene group or a straight alkenylene group; $R^2$ is hydrogen atom or phenyl group; Y is a straight alkylene group, a straight alkenylene group or a straight alkynylene group; $R^3$ and $R^4$ are different from each other and when one is hydrogen atom, another is a lower alkoxy group; $R^5$ is hydrogen atom; $R^6$ and $R^7$ are hydroxy groups; $R^8$ and $R^9$ are different from each other, and are either hydrogen atom or hydroxy group.

5. The compound according to Claim 4, wherein $R^1$ is hydrogen atom or phenyl group; X is a straight alkylene group; $R^2$ is hydrogen atom; Y is a straight alkylene group, a straight alkenylene group or a straight alkynylene group; $R^3$, $R^5$ and $R^8$ are hydrogen atoms; $R^4$ is a lower alkoxy group; and $R^6$, $R^7$ and $R^9$ are hydroxy groups.

6. The compound according to Claim 5, wherein $R^1$ is phenyl group; X is tetramethylene group; $R^2$ is hydrogen atom; Y is heptadecamethylene group; $R^3$, $R^5$ and $R^8$ are hydrogen atoms; $R^4$ is methoxy group; and $R^6$, $R^7$ and $R^9$ are hydroxy groups.

7. The compound according to Claim 5, wherein $R^1$ is phenyl group; X is tetramethylene group; $R^2$ is hydrogen atom; Y is heptamethylene group; $R^3$, $R^5$ and $R^8$ are hydrogen atoms; $R^4$ is methoxy group; and $R^6$, $R^7$ and $R^9$ are hydroxy groups.

8. The compound according to Claim 5, wherein $R^1$ is hydrogen atom; X is heptamethylene group; $R^2$ is hydrogen atom; Y is heptadecamethylene group; $R^3$, $R^5$ and $R^8$ are hydrogen atoms; $R^4$ is methoxy group; and $R^6$, $R^7$ and $R^9$ are hydroxy groups.

9. The compound according to Claim 5, wherein $R^1$ is hydrogen atom; X is octadecamethylene group; $R^2$ is hydrogen atom; Y is undecamethylene group or nonamethylene group; $R^3$, $R^5$ and $R^8$ are hydrogen atoms; $R^4$ is methoxy group; and $R^6$, $R^7$ and $R^9$ are hydroxy groups.

10. The compound according to Claim 5, wherein $R^1$ is phenyl group; X is tetramethylene group; -Y-$R^2$ is 9-decenyl group; $R^3$, $R^5$ and $R^8$ are hydrogen atoms; $R^4$ is methoxy group; and $R^6$, $R^7$ and $R^9$ are hydroxy groups.

11. The compound according to Claim 5, wherein $R^1$ is hydrogen atom; X is octadecamethylene group; -Y-$R^2$ is 9-decynyl group; $R^3$, $R^5$ and $R^8$ are hydrogen atoms; $R^4$ is methoxy group; and $R^6$, $R^7$ and $R^9$ are hydroxy groups.

12. The compound according to Claim 5, wherein $R^1$ is hydrogen atom; X is hexadecamethylene group, heptadecamethylene group or nonadecamethylene group; $R^2$ is hydrogen atom; Y is undecamethylene group; $R^3$, $R^5$ and $R^8$ are hydrogen atoms; $R^4$ is methoxy group; and $R^6$, $R^7$ and $R^9$ are hydroxy groups.

13. A process for preparing an acylaminosaccharide compound represented by the formula (I):

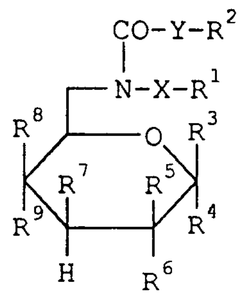

(I)

wherein $R^1$ represents hydrogen atom, or an aryl group or a heterocyclic group which may have a substituent; X represents a single bonding arm, an alkylene group, an alkenylene group or an alkynylene group; $R^2$ represents (1) hydrogen atom, (2) an aryl group or a heterocyclic group which may have a substituent, (3) an aryl lower alkylcarbamoyl group which may have a substituent, (4) carbonyl group which is substituted by a group obtained by eliminating a hydrogen atom from amino group of an amino acid or an ester thereof, or (5) carboxyl group which may be esterified; Y represents a single bonding arm, an alkylene group which may have a cycloalkylene group at an end thereof, an alkenylene group, an alkynylene group or a tricycloalkylene group; $R^3$ and $R^4$ are different from each other, and when one represents hydrogen atom, another represents a lower alkoxy group, or substituted or unsubstituted phenoxy group; $R^5$ and $R^6$ are different from each other, and represent either hydrogen atom or hydroxy group; and when $R^7$ represents hydroxy group, $R^8$ and $R^9$ are different from each other and represent either hydrogen atom or hydroxy group, or when $R^7$ and $R^8$ are bonded to each other at ends thereof to form a lower alkylenedioxy group, $R^9$ represents hydrogen atom,
or a pharmaceutically acceptable salt thereof, which comprises reacting an animosaccharide compound represented by the formula (II):

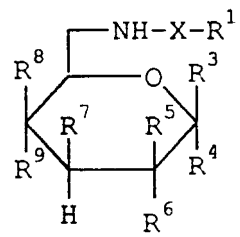

(II)

wherein the symbols have the same meanings as above, or a salt thereof, with a carboxylic acid compound represented by the formula (III):

$R^2$-Y-COOH (III)

wherein $R^2$ and Y have the same meanings as above, a salt thereof or a reactive derivative thereof.

**14.** The process according to Claim 13, wherein the reaction is carried out in the presence of a dehydrating agent in a solvent at -20 °C to 70 °C.

**15.** The process according to Claim 13, wherein said reaction is carried out in the presence or absence of an acid acceptor in a solvent at -20 °C to 70 °C.

**16.** A pharmaceutical composition which comprises a therapeutically effective amount of the compound as set forth in Claim 1 in admixture with a conventional pharmaceutically acceptable carrier or diluent.

**17.** The use of the compound according to Claim 1, which is used as an infection-preventive agent or an anti-infectious property enhancing agent.

European Patent Office

# EUROPEAN SEARCH REPORT

Application Number
EP 93 30 8624

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | CHEMICAL AND PHARMACEUTICAL BULLETIN vol. 31, no. 11 , November 1983 , TOKYO JP pages 3924 - 3930 T. MORIKAWA ET AL 'A new class of nitrosoureas. IX. Synthesis and antitumor activity of 3-substituted 1-(2-chloroethyl )-3-(methyl-alpha-D-glucopyranosid-6-yl or methyl 2-acetamido-2-deoxy-alpha-D-glucopy ranosid-6-yl)-1-nitrosoureas' * the whole document * --- | 1,13,16, 17 | C07H15/203 C07H15/04 A61K31/70 C07H15/10 C07H15/18 C07H15/26 C07H17/02 C07H17/06 |
| A,D | CARBOHYDRATE RESEARCH vol. 45 , 1975 , AMESTERDAM, NL pages 65 - 72 T. D. INCH ET G. J. LEWIS 'Use of carbohydrate derivatives for studies of phosphorus stereochemistry. Part VI. A stereochemical comparison of S-alkyl alkylphosphonothioate-sodium methoxide and S-alkyl phosphorothioate-sodium methoxide reactions.' * page 67; example 12 * ----- | 1 | |
| | | | TECHNICAL FIELDS SEARCHED (Int.Cl.6) |
| | | | C07H A61K |

The present search report has been drawn up for all claims

| Place of search | Date of completion of the search | Examiner |
|---|---|---|
| THE HAGUE | 30 March 1994 | Moreno, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)